# EUROPEAN PATENT SPECIFICATION

(11) **EP 0 529 645 B1**
(45) Date of publication and mention of the grant of the patent: **23.10.1996**
(21) Application number: 92114713.8
(22) Date of filing: 04.09.1987
(51) Int. Cl.: C07C 331/28, C07C 229/16, C07C 211/15

(54) **Process for the preparation of backbone polysubstituted chelates**
Verfahren zur Herstellung hauptkettenpolysubstituierter Chelate
Procédé de préparation de chélates polysubstitués sur leur chaine principale

(30) Priority: 05.09.1986 US 903723
(43) Date of publication of application: 03.03.1993
(62) Divisional of application: 87906504.3
(73) Proprietor: GANSOW, Otto A., Washington, DC 20008 (US); Brechbiel, Martin W., Annandale, Virginia 22003-1155 (US)
(72) Inventor: GANSOW, Otto A., Washington, DC 20008 (US); Brechbiel, Martin W., Annandale, Virginia 22003-1155 (US)
(74) Representative: Perry, Robert Edward

(56) References cited:
- US-A- 4 454 106
- US-A- 4 472 509
- US-A- 4 622 420

## Description

This invention relates to a method for preparing backbone polysubstituted chelates that are suitable for the formation of metal chelates and metal-chelate protein conjugates.

Interest in the art in metal chelates and in methods for forming metal chelate-protein conjugates for diagnostic and therapeutic purposes continues. Representative type chelates and conjugates and methods for forming conjugates are disclosed, inter alia, in U.S. Patents 4,454,106, 4,472,509, 4,339,426. One example of such conjugates is a metal chelate-monoclonal antibody conjugate. Other proteins including antibody fragments, polyclonal antibodies, antigens, blood proteins, or proteins bound to blood lymphocytes or other cells can also be employed in the formation of conjugates.

A method for synthesis of bifunctional metal chelates for conjugation to proteins involves reduction of amino acid amides to ethylenediamines to form monosubstituted derivatives which are converted to bifunctional ethylenediaminetetraacetic acid (EDTA) chelates by alkylation with haloacetic acid. (Yeh, et al., Anal. Biochem., 100:152, 1979). Similarly,monosubstituted diethylenetriamine is synthesized by reaction of ethylenediamine with an amino acid ester and reduction of the resulting amide carbonyl. (Brechbiel, et al. Inorg. Chem., 25:2772-2781 (1986). Alkylation of the diethylenetriamine with haloacetic acid produces a monosubstituted bifunctional diethylenetriamine- pentaacetic acid (DTPA) chelate.

Another method of synthesis of a bifunctional DTPA involves reaction of a DTPA or EDTA carboxylate with an chloroformate ester to form a reactive anhydride. (Krejcarek, et al., Biochem. Biophys Res. Commun., 77: 581, 1977). The dianhydride of DTPA used as a bifunctional chelate is prepared by dehydration of the parent DTPA. (Hnatowich, et al., Int. J. Appl. Rad. Isot., 33:327, 1982). The practice of using an EDTA chelate monosubstituted at the carbon-1 position to better retard the release of metal from chelate in vitro than the unsubstituted EDTA chelate has also been reported. (Meares, et. al., Anal. Biochem., 142:68, 1984).

Generally, the prior art has formed metal-protein chelate conjugates by mixing monosubstituted bifunctional EDTA or DTPA chelates or DTPA anhydrides with proteins followed by reaction with the metal to be chelated. (Krejcarek, et al., Biochem. Biophys. Res. Commun., 77: 581, 1977); Brechbiel, et al., Inorg. Chem., 25:5783, 1986). Imaging of tumor target sites in vivo with metal chelate conjugated monoclonal antibodies prepared according to these methods has been reported. (Khaw, et al., Science, 209:295, 1980). Sheinberg, et al., Science, 215:1511, 1982). Diagnosis of human cancer in vivo using metal chelate conjugated monoclonal antibody has also been reported. (Rainsbury, et al., Lancet, 2:694, 1983). But attempts to employ the tumor localizing properties of metal chelate conjugated monoclonal antibodies for therapeutic purposes have not found common usage, in part because metals may be (and often are) released from the metal chelate conjugate in vivo and, particularly in the case of radioactive metal salts, may produce undesirable concentrations of toxic radionuclides in bone marrow or the like even if the conjugates are rigorously purged of adventitiously bonded metal. A process for purifying metal chelate protein conjugates of adventitiously bonded metals is disclosed in U.S. Patent 4,472,509. The importance of using very strong metal chelates to firmly link radiometals to monoclonal antibodies and of rigorous purification of the conjugates to effect maximal tumor localization and minimize delivery to non-target tissues is discussed in Brechbiel, et al. (Inorg. Chem., 25, 1986). Undesirable localization of potentially therapeutic radionuclides released in mice in vivo from metal chelate conjugated polyclonal antibodies have precluded therapy investigation in humans. (Vaughn, et. al., EIR-Bericht. 78, 1986). Increased in vivo bone uptake of radiometal injected for therapy as a metal chelate conjugated monoclonal antibody has also been reported. (Hnatowich, et al., J. Nucl. Med, 26:503, 1985). The amount of potentially therapeutic doses in humans of radiometal chelated polyclonal antibody has been limited by bone marrow toxicity Order, et al., Int. J. Rad. Oncol., 12: 277, 1986).

It is evident from the above that there continues to be a need for more effective metal chelate protein conjugates that firmly link metals to proteins to minimize metal release and permit highly selective delivery of metals to targeted sites in vivo.

According to the present invention, a method for preparing polysubstituted diethylenetriaminepentaacetic acid chelate comprises the sequential steps of:
(a) reacting an amino-protected alpha amino acid with an amino acid amide;
(b) deprotecting the protected amino acid;
(c) reducing the product obtained in step (b) to produce polysubstituted diethylenetriamine;
(d) converting said diethylenetriamine to desired polysubstituted diethylenetriaminepentaacetic acid or an ester thereof by alkylating with a haloacetic acid or haloacetate ester.

### DESCRIPTION OF THE INVENTION

The present invention is now described with reference to the following drawings, each of which illustrate a reaction scheme for the preparation of compounds of Formula 1, wherein:

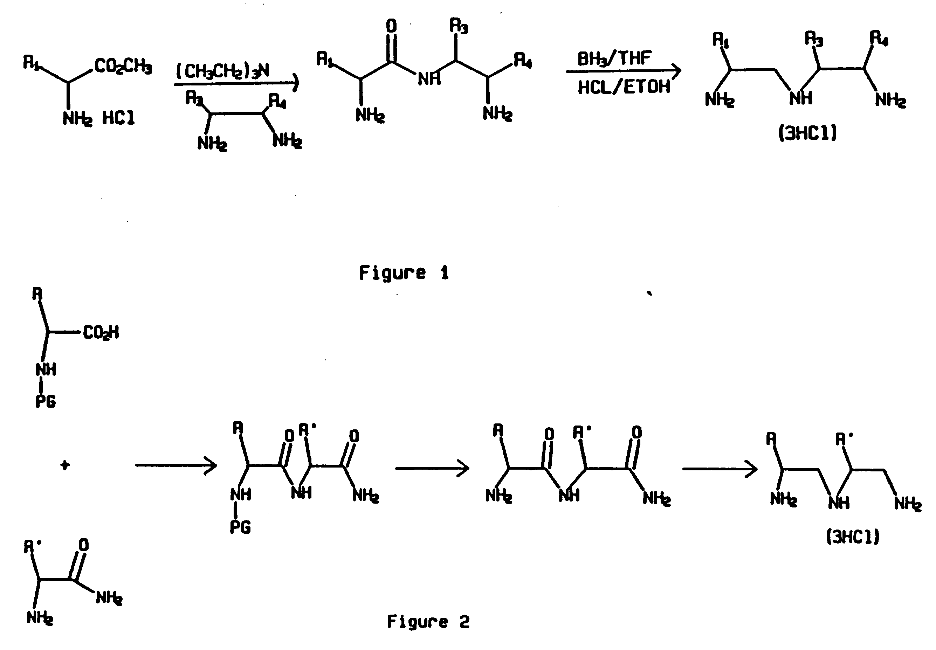

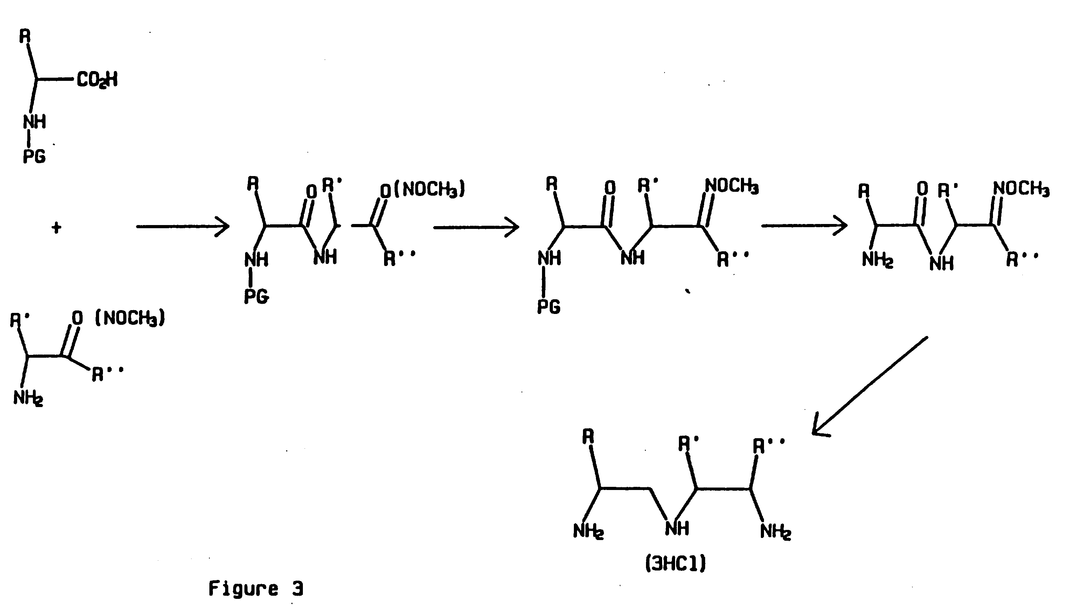

Fig. 1 shows a scheme for preparation of a polysubstituted diethylenetriamine, where R₁ is para-nitrobenzyl and R_{3,4} are aryl/alkyl groups as described in the text. In the case where R_{3,4} are the methyl group, the diethylenetriamine product of the scheme is compound (d) of Table 1.

Fig. 2 shows a scheme for preparation of polysubstituted diethylenetriamine, where PG represents a protecting group (vide infra), R and R' are para-nitrobenzyl or aryl/alkyl groups, respectively, as described in the text. In the case where R is para-nitrobenzyl and R' methyl, the product diethylenetriamine is compound (a) of Table 1. In the case where R is methyl and R' is para-nitrobenzyl, the product diethylenetriamine is compound (c) of Table 1.

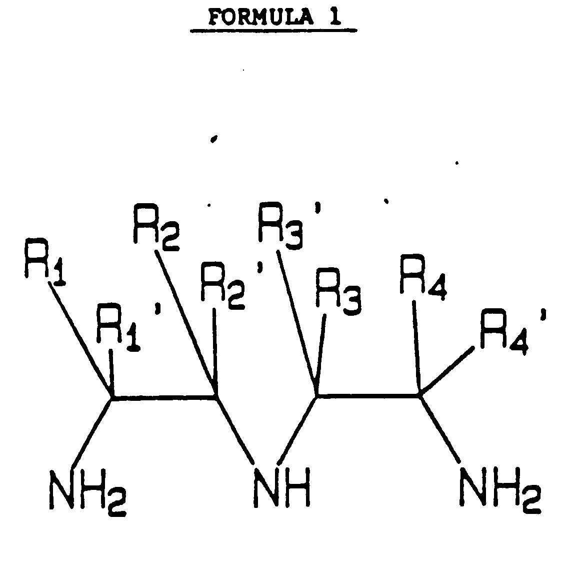

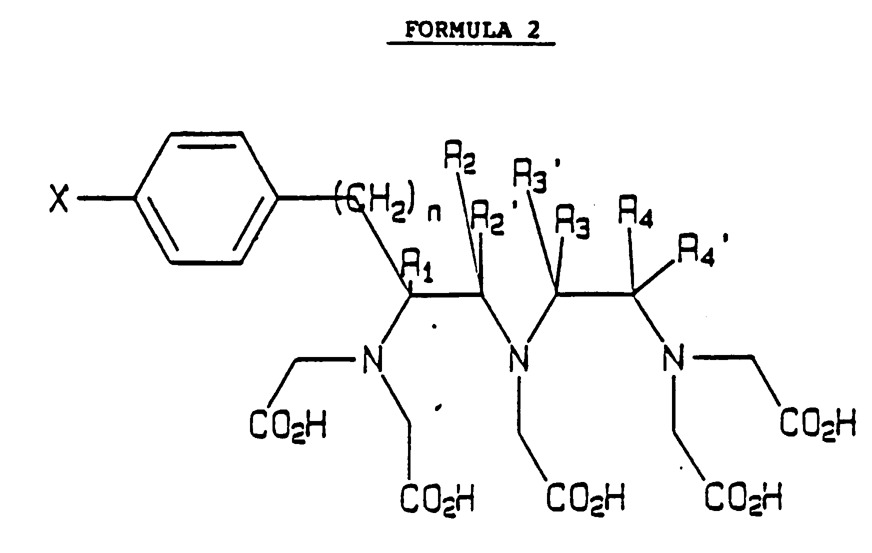

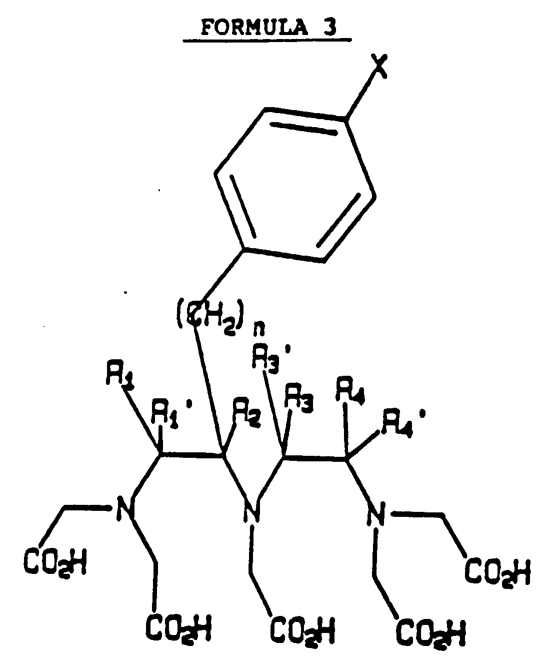

**Table 1**

| The following substituents refer to Formula 1. | | | | |
|---|---|---|---|---|
| | R₁,R₁' | R₂,R₂' | R₃,R₃' | R₄,R₄' |
| 1 (a) | SCNBz, H | H, H | CH₃, H | H, H |
| 1 (b) | SCNBz, H | H, H | H, H | CH₃, H |
| 1 (c) | H, H | SCNBz, H | H, H | CH₃, H |
| 1 (d) | SCNBz, H | H, H | CH₃, H | CH₃, H |

wherein SCNBz is para-isothiocyanatobenzyl

**Table 2**

| The following substituents relate to Formulas 2 and 3. | | | | |
|---|---|---|---|---|
| | R₁,R₁' | R₂,R₂' | R₃,R₃' | R₄,R₄' |
| 2 (a) | SCNBz, H | H, H | CH₃, H | H, H |
| 2 (b) | SCNBz, H | H, H | H, H | CH₃, H |
| 3 (c) | H, H | SCNBz, H | H, H | CH₃, H |
| 2 (d) | SCNBz, H | H, H | CH₃, H | CH₃, H |

wherein SCNBz is para-isothiocyanatobenzyl

The introduction of reactive side chains into the carbon backbone structure of chelates has been described in the prior art. (Meares, et al. 142 Anal. Biochem. 68, 1984).

Essentially all syntheses of DTPA have as their penultimate reaction step the alkylation of a parent diethylenetriamine. Thus the methods for preparation of carbon backbone polysubstituted DTPA reduces to the preparation of the parent diethylenetriamines.

The conventional method for preparation of a substituted diethylenetriamine is illustrated in Fig. 1. The process consists of reaction of an amino acid ester with an ethylenediamine followed by reduction to the diethylenetriamine.

The reactions of Fig. 1 can be used to provide a compound 1(d) of Table 1 wherein R₁ is para-nitrobenzyl and R₃, R₄ are methyl. In accordance with this scheme, 2, 3-diaminobutane may be reacted with p-nitrobenzylalanine methyl ester and the product reduced with diborane to provide the parent diethylenetriamine of 2(d). To produce 2(d), the nitrogens of the parent diethylenetriamine are alkylated with bromoacetic acid, the nitro group is then catalytically reduced with hydrogen, and the resulting amine reacted with thiophosgene.

However, if the method of Fig. 1 is used to prepare the compounds 2(a) and/or 2(b) of the Table 2, the result of the reaction of 1, 2-diaminopropane with p-nitrophenylalanine methyl ester, followed by reduction, alkylation, reduction and thiocarbonylation steps of the paragraph above, results in a mixture of compounds 2(a) and 2(b) which are geometric isomers. Separation of the isomers is not practicable by currently available methods. No modifications of the method of Fig. 1 can be used to prepare isolated, pure samples of 2(a) and 2(b).

Since pure samples of 2(a) and 2(b) are required for pharmaceutical use, the novel process shown in Fig. 2 for preparation of the parent diethylenetriamine of the polysubstituted DTPA were devised.

According to the process of Fig. 2, to prepare compound (a), an amino-protected alpha amino acid, in this case t-butyloxycarbonyl-p-nitrophenylalanine is coupled to an amino acid amide, in this case, alanine amide, by activating the carboxylate with a suitable reagent. Such reagents are known in the art and may include inter alia the preferred 1, 3-dicyclohexylcarbodiimide or other carbodiimides, carboxycarbonic esters, mixed anhydrides, and the like. The coupled product is next deprotected with trifluoroacetic acid or other deprotecting reagent, then reduced with diborane to yield the parent diethylenetriamine of 2(a). The standard alkylation, reduction and thiocarbonylation steps provide geometrically pure 2(a). Compound 3(c) can be prepared via an analogous route.

The present invention is illustrated by the following Example:

### Example

Preparation of 1-p-isothiocyantobenzyl)-3-methyl DTPA. (Synthesis of compound 2(a) in Table 1 as a single geometric isomer).

### t-Butoxycarbonyl-(d,1)-p-nitrophenylalanine:

p-Nitrophenylalanine (7.94 g, 37.8 mmol) was dissolved in 50% aqueous dioxane solution (60 ml) and triethylamine (7.9ml, 56.7 mmol) added [2-(t-butoxycarbonyloxyamino)2-phenylacetonitrile]. (10.24 g, 41.6 mmol, Aldrich Chemical Co.) was added and the solution stirred for two hours. Ethyl acetate (100 ml) and H₂O (50 ml) were added and the contents poured into a separatory funnel. The aqueous layer was retained and extracted twice with ethyl acetate (100 ml). The aqueous layer was cooled to 0°C and the pH was adjusted to 2.0 with 3N HCl, whereupon a precipitate formed which was collected and dried under vacuum. The filtrate was extracted with ethyl acetate twice (100 ml), dried over MgSO₄and stripped to dryness. The two fractions proved to be identical and were combined (11.0 g, 94.0%). The melting point of the compound was 165°C.

¹H NMR (220 MHz, DMSO-d₆) 8.036 (d,2,J=8.00), 7.29 (d,2,J=8.00), 5.38 (d,1,J=8.00), 4.44 (m,1),3.25 (dd,1,J=13.0,6.00), 3.05 (dd,1,J=13.0,6.00), 1.39 (s,9); CI-MS 311 ((M+1)/z).

### t-Butoxycarbonyl-(d1)-p-nitrophenylalaninyl-(1)-alanine amide:

BOC-(d1)-p-nitrophenylalanine (10.0 g, 32.26mmol), 1-alanine amide hydrobromide (5.45 g, 32.26mmol), triethylamine (4.487 ml, 32.36 mmol), and 1-hydroxybenzotriazole (3.84 g, 28.4 mmol) were dissolved in ethyl acetate (400 ml). Dicyclohexylcarbodiimide (7.30 g, 35.44 mmol) in ethyl acetate (25 ml) was added and the reaction mixture was allowed to stir for 18 hours after which three drops (about 0.15 ml) of concentrated acetic acid were added. The dicyclohexylurea was filtered off and the filtrate was extracted sequentially with saturated sodium chloride salt solution (100 ml), 1N HCl (3 x 100 ml), saturated salt solution (100 ml), 5% NaHCO₃(3 x 100 ml), and saturated salt solution (100 mL). The organic solution was dried over MgSO₄, filtered, and reduced to 50 ml. Petroleum ether (50 mL) was added and the contents of the flask were cooled to 0°C for 12 hours. The precipitate was collected on a Buchner funnel and dried under vacuum (10.55 g, 86.1 %).

¹H NMR (220 MHz, CDCl₃/d₆-DMSO) 8.08 (d,2,J=9.0), 7.91 (m,1), 7.45 (d,2,J=9.0), 7.14 (d,1,J=12.0), 6.68 (m,2), 4.35 (m,2), 3.17 (dd,1,J=15.0, 6.0), 2.98 (dd,1.J=15.0, 8.0), 1.38 (s,9); CI-MS 381 ((M+1)/z). Anal. Calcd. for C₁₇H₂₄N₄O₆: C, 53.68; H, 6.31; N, 14.73. Found: C, 53.92; H, 6.59; N, 14.84.

### 2-Methyl-4-(p-nitrobenzyl)diethylenetriamine trihydrochloride:

The dipeptide amide described above (5.10 g, 13.42 mmol) was deprotected by treatment with trifluoroacetic acid (50 ml) for one hour after which the solution was rotary evaporated to near dryness. Methanol (50 ml) was added and the solution was taken to dryness. The resulting solid was held at .01 mm and 50°C for 8 hours to insure removal of the residual acid.

The resulting ammonium salt (5.10 g, 13.42 mmol) in THF (50 ml) was added to a flame dried 250 ml three neck flask fitted with a condenser under argon atmosphere. The flask was cooled to 0°C and 1M BH₃THF (30.8 ml) was added via syringe. The reaction solution was heated to a vigorous reflux for two hours and then allowed to stir at room temperature for an additional two hours. The reaction flask was cooled to 0°C and methanol (25 ml) was slowly injected to decompose excess hydride. The solution was reduced to dryness, taken up in absolute ethanol (50 ml), and concentrated HCl (50 ml) was added. The solution was vigorously refluxed for two hours and then stripped to dryness. The residue was dissolved in H₂O, loaded onto a 1.5 x 20 cm AG50W X8, H+ form, ion exchange column, and washed with H₂O until the eluant was neutral. The product was eluted from the column with concentrated HCl (125 ml), concentrated to 10 ml, and lyophilized overnight. The remaining solid was found to be substantially pure (1.823 g, 66.2%).

¹H NMR (500 MHz, D₂O, pH 1.0) 8.268 (d,2,J=8.0), 7.614 (d,2,J=8.0), 4.106 (m,1), 3.773 (m,1), 3.680-3.450 (m,3), 3.393 (m,1), 3.312 (m,1), 3.212 (m,1),1.493 (br. t,3); (500 MHz, D₂O, pH 11.0) 8.091 (d,2,J=8.0), 7.438 (d,2,J=8.0), 3.167 (m,1), 2.910 (m,1), 2.75-2.45 (overly complicated multiplet, 6), 1.031 (br. s, 3); CI-MS 253 ((M+1)/s). Anal, Calcd. for C₁₂H₂₃N₄O₂Cl₃: C, 39.85; H, 6.36; N, 15.49. Found: C, 39.88; H, 6.36; N, 15.28.

Conversion of this diethylenetriamine to compound 2(a) was achieved by the method described in the Reference Example, below.

### Reference Example

Preparation of 1,(2)-methyl-4-p-isothiocyanato benzyl)diethylenetriaminepentaacetic acid. (The mixture of geometric isomers of compounds 2(a), 2(b) in Table 2.

### Methyl p-nitrophenylalanine hydrochloride

Dry methanol (200 ml) was saturated with HCl(-g) in a two-necked round bottom flask cooled to -10°C. p-Nitrophenylalanine (10.0 g, 47.6 mmol) was added in one portion and left to stir for about 18 hours, the solution was evaporated to near dryness on a rotary evaporator and the precipitated product collected in a Buchner funnel. After drying under vacuum at about 50°C, the yield was 10.97 grams (88.3%). A TLC (thin layer chromatography) of the free amino ester run in CHCl₃:MeOH (4:1) revealed an R_{f}= 0.85-0.88. ¹H NMR (220 MHz, D₂O, pH 1.5) 8.20 (d,2 J=10.0), 7.53 (d,2,J=10.0), 4.55 (t,1,J=5.00), 3.84 (s,3), 3.43 (m,2); CI-MS 225 ((M+1)/z). Anal. Calcd. for C₁₀X₁₃N₂O₄Cl: C, 46.07; H, 5.03; N, 10.74; Cl, 13.60. Found: C, 45.87; H, 5.08; N, 10.48; Cl, 13.58.

### N-(2-amino-[1(2)-methyl]ethyl)-p-nitrophenylalanine amide

Methyl p-nitrophenylalanine hydrochloride (9.80 g, 37.63 mmol) was treated with triethylamine (6.78 ml, 45.2 mmol) to liberate the amino ester. After removal of the solvent, the residual oil was then added dropwise in methanol (5 ml) to 1,2-diaminopropane (50 ml) at room temperature (20°-24°C) while vigourously stirring. After stirring 18 hours, the excess solvent was removed via rotary evaporation at 50°C at .01 mm of vacuum until a constant weight was achieved (10.01 g, 96%). TLC of the product in CHCl₃:MeOH (4:1) on silica revealed an R_{f}=0.10-0.12.

¹H NMR (220 MHz, D ), pH 10.0) 8.06 (d,2,J=7.5), 7.41 (d,2,J=7.5), 3.72 (t,1,J=8.0), 3.18-2.73 (m,5), 0.918 (m,3); CI-MS 267 ((M+1)/z). Anal. Calcd. for C₁₂H₁₈N₄O₃: C, 54.53; H, 6.77; N, 21.05. Found: C, 54.33; H,6.76; N, 20.92.

### 1(2)-Methyl-4-(p-nitrobenzyl)diethylenetriamine trihydrochloride

N-(2-amino-[1(2)-methyl]ethyl)-p-nitrophenylalanine amide (9.90 g, 37.2 mmol) was reduced with 1M (boronhydride tetrahydrofuran) BH₃THF (200 mL). A one liter three-neck round bottom flask was fitted with a reflux condenser, septum, argon inlet and bubbler exit, and flame dried. The amide (8.12 g, 38.9 mmol) was washed into the reaction flask with dry tetrahydrofuran THF (150 ml) and cooled to -10°C. Next, 1 M BH₃THF solution (200 ml) was added with a syringe. The reaction solution was stirred one hour at -10°C, then raised to a gentle reflux for 18 hours, after which it was cooled to -10°C and dry methanol (25 ml) was injected. The solution was brought to room temperature and stripped to near dryness. Methanol (25 ml) was again added and the solution evaporated to near dryness. Cleavage of the borane aggregate required a vigorous reflux of the HCl saturated ethanolic solution plus the addition of concentrated aqueous HCl (5ml). The product precipitated cleanly and after cooling to 0°C for 6 hours was collected and dried under vacuum (11.60 g, 86.3%). CI-MS 362 ((M+1)/z). Anal. Calcd. for C₁₂H₂₃N₄O₂Cl₃: C, 39.85; H, 6.65; N, 15.49. Found: C, 39.99; H, 6.64; N, 15.14.

1(2)-Methyl-4-(p-nitrobenzyl)diethylenetriaminepentaacetic acid: The parent diethylenetriamine (1.0 g, 2.77 mmol) was treated with bromoacetic acid (5.767 g, 41.5 mmol) and 7N KOH (13.04 ml). The reaction solution was allowed to stir for 72 hours at room temperature. The solution was acidified to pH 1.5 with concentrated HBr and extracted with ether (3 X 100 ml). The aqueous solution was evaporated to a solid and loaded onto a 2.6 x 30cm ion exchange column AG50W X 8, 200-400 mesh, H+ form, (BioRad Inc., Richmond CA) and washed with H₂O to remove the unreacted materials, hydrolysis products and salts. The crude product was eluted with 2N aqueous NH₃. The crude product was further purified by HPLC using a 10 x 250 mm, C₁₈ reverse phase column with a 25 minute gradient of aqueous .05M triethylammonium acetate to 100% methanol at a flow rate of 3 ml/min. The product had a retention time of 9.1 minutes. The combined fractions from the HPLC were re-chromatographed on an AG50W X 8 column as specified above to remove the triethylammonium acetate buffer. The product was collected and the solvent evaporated to a solid.
(.648 g, 43.2%).

1(2)-Methyl-4-(p-aminobenzyl)diethylenetriaminepentaacetic acid The parent nitrobenzyl DTPA (100.0 mg, 0.1845 mmol) was hydrogenated with Pd/C. A water-jacketed three-neck flask (50 ml) was charged wtih 10% Pd/C(43mg), H₂O (5ml) and a stirring bar. The center neck was attached to an atmospheric hydrogenation apparatus, one side neck was fitted with an injection valve, and the remaining neck was firmly stoppered. The assembled hydrogenation apparatus was evacuated and flushed with hydrogen while the reaction flask was cooled to 4°C. The nitrocompound was dissolved in H₂O (10ml) and 5M NaOH was added to bring the pH to 10.0. The solution was injected into the reaction flask and hydrogen uptake monitored. After the reaction, the mixture was filtered through a fine frit with Celite 535 (Fluka AG, Switzerland). The solvent was removed and the residue dried under vacuum for 18 hours with yield being essentially quantitative.

1(2)-Methyl-4-(p-isothiocyanatobenzyl)diethylenetriamine pentaacetic acid (The mixture of compounds, a,b in Table 2.) The parent mixture of aniline precursors to compound 2(a), 2(b) and described above (0.095 g, 0.1845 mmol) was dissolved in H₂O (5ml) pH 8.5 and converted to a crude product by treatment with thiophosgene (0.212 g, 1.845 mmol) in CHCl₃(10 ml). The crude product was purified by column chromatography on a 1 x 30 cm Florisil (Sigma, St. Louis, Mo) column eluted with CH₃CN:H₂O (30:8) with the product eluting first. The solvent was removed with minimum heating and the remaining aqueous solution lyophilized overnight. The R_{f} of the product on silica using CH₃CN:H₂O (30:8) was 0.20. The IR spectrum possessed the characteristic absorption at 2100 cm⁻¹for the isothiocyanate.

## Claims

1. A method for preparing polysubstituted diethylenetriaminepentaacetic acid chelate comprising the sequential steps of :
(a) reacting an amino-protected alpha amino acid with an amino acid amide;
(b) deprotecting the protected amino acid;
(c) reducing the product obtained in step (b) to produce polysubstituted diethylenetriamine;
(d) converting said diethylenetriamine to desired polysubstituted diethylenetriaminepentaacetic acid or an ester thereof by alkylating with a haloacetic acid or haloacetate ester.

## Patentansprüche

1. Verfahren zur Herstellung von polysubstituiertem Diethylentriaminpentaessigsäurechelat, gekennzeichnet durch die aufeinanderfolgenden Stufen:
(a) Umsetzung einer aminogeschützten alpha-Aminosäure mit einem Aminosäureamid;
(b) Freisetzung der Schutzgruppe der geschützten Aminosäure;
(c) Reduktion des in Stufe (b) erhaltenen Produkts zur Erzeugung von polysubstituiertem Diethylentriamin;
(d) Umwandlung dieses Diethylentriamins zur gewünschten polysubstituierten Diethylentriaminpentaessigsäure oder einem Ester davon durch Alkylieren mit einer Halogenessigsäure oder einem Halogenacetatester.

## Revendications

1. Méthode de préparation d'un chélate d'acide diéthylènetriaminepentaacétique polysubstitué comprenant les étapes séquentielles de :
(a) faire réagir un acide alpha aminé amino-protégé avec un amide d'acide aminé ;
(b) supprimer la protection de l'acide aminé protégé ;
(c) réduire le produit obtenu à l'étape (b) pour donner la diéthylènetriamine polysubstituée ;
(d) convertir ladite diéthylènetriamine en acide diéthylènetriaminepentaacétique polysubstitué souhaité ou son ester par alkylation avec un acide haloacétique ou un ester d'haloacétate.
